# EUROPEAN PATENT SPECIFICATION

(11) **EP 0 823 261 B1**
(45) Date of publication and mention of the grant of the patent: **21.05.2003**
(21) Application number: 97305887.8
(22) Date of filing: 04.08.1997
(51) Int. Cl.: A61M 25/09

(54) **Guidewire having a distal tip that can change its shape within a vessel**
Führungsdraht mit distaler Spitze mit im Blutgefäss veränderbarer Gestalt
Bout de fil de guidage avec la capacité de changer sa forme dans un vesseau sanguin

(30) Priority: 05.08.1996 US 693378
(43) Date of publication of application: 11.02.1998
(73) Proprietor: Cordis Corporation, Miami Lakes Florida 33014 (US)
(72) Inventor: Lorenzo, Juan A., Davie, Florida 33328 (US); Barbre, Carol, Miami Lakes, Florida 33014 (US)
(74) Representative: Mercer, Christopher Paul

(56) References cited:
- EP-A- 0 363 661
- EP-A- 0 480 427
- EP-A- 0 646 364
- EP-A- 0 739 641
- EP-A- 0 778 043
- US-A- 5 143 085

## Description

The present invention relates to steerable guidewires for introducing medical catheters, such as balloon catheters, within the vasculature of patients. The present invention has even further relation to such a guidewire which can change the shape of its distal tip without being removed from the patient.

### BACKGROUND OF THE INVENTION

Percutaneous transluminal coronary angioplasty (PTCA) is a therapeutic medical procedure used to increase blood flow through the coronary artery and can often be used as an alternative to coronary by-pass surgery. An elongated catheter having a deflated balloon at its distal end is guided through a patient's cardiovascular system to the coronary artery of the heart. The balloon is inflated to compress deposits that have accumulated along the inner walls of the coronary artery to widen the artery lumen and increase blood flow. Typically, the balloon catheter is guided to the specific area within the vessel by an elongated guidewire. The guidewire is inserted into the patient and routed through the cardiovascular system and can be viewed on an x-ray imaging screen.

The path the guidewire follows during this procedure is often tortuous. The distal tip of the guidewire is flexible to avoid damaging inner walls of the blood vessels that the guidewire tip contacts along the tortuous path. The distal tip is often pre-bent to a desired configuration so that the guidewire can be inserted into branching blood vessels along its path. When the tip is pre-bent, the physician must be able to orient the tip so it can be pushed into these branching blood vessels. Examples of prior art guidewires are shown in U.S. Patent 4,846,186 issued to Box et al. on July 11, 1989 and U.S. Patent 5,267,574 issued to Viera et al. on December 7, 1993, both of which are hereby incorporated herein by reference. Additional guidewire assemblies are disclosed in U.S. Patent 5,143,055 and European Patent Application 0,363,661. The former is controlled by providing a part that is formed of a heat activated memory alloy. The latter is controlled by providing a pre-bent tip and a stiff rod for guiding along the tip for straightening the tip.

Such guidewires typically have a core made from stainless steel or the like and coated with a lubricity enhancing agent, such as Teflon ®. The distal end of the guidewire is not coated as such and usually comprises one or two tapered portions which reduce the diameter of the core wire at its distal end. The distal most portion of the core wire is then flattened to form a ribbon tip which makes it easier for a physician to form into a desired shape. A flexible coiled wire spring surrounds the distal tip of the core wire and is attached thereto. The coil separates from the core wire for a predetermined length and is attached proximal to the flattened distal portion of the core wire.

When the physician is navigating the tortuous paths of the human vasculature, it is often desirable to have the distal tip of the guidewire bent to a particular shape. This aids the guidewire in making turns into branching vessels or the like. However, during the same procedure, the physician may often want the distal tip of the guidewire to be flexible, not having a pre-bent configuration. This needs to be accomplished without removing the guidewire from the patient. Therefore, there has been a desire to have a guidewire whose tip shape can change without being removed from the body of a patient. The present invention fulfills such a desire.

### SUMMARY OF THE INVENTION

In accordance with the present invention there is provided a guidewire as defined in claim 1 for navigating through body vessels. The guidewire has a proximal end, a distal end and a longitudinal axis extending therebetween. The guidewire has an outer tube with proximal and distal ends. The outer tube is made from a material exhibiting substantially no shape memory retention. The guidewire includes a flexible distal tip attached to the distal end of the outer tube and extending distally thereto. The guidewire further includes a core wire having distal and proximal ends. The distal end of the core wire is made from a material exhibiting shape memory retention. The core wire can slide longitudinally within the tube. The core wire slides between a retracted position, where the distal end of the core wire is proximal to the flexible distal tip and takes the shape of the outer tube, and an extended position, wherein the distal end of the core wire is distal to the distal end of the outer tube so that it can return to a preformed shape.

### BRIEF DESCRIPTION OF THE DRAWINGS

While the specification concludes with claims which particularly point out and distinctly claim the subject matter forming the present invention, it is believed that the invention will be better understood from the following description of the preferred embodiment taken in conjunction with the accompanying drawings wherein:
Figure 1 is a simplified cross-sectional view of a guidewire made in accordance with the present invention.
Figure 2 is similar to that of Figure 1 but showing the distal tip of the guidewire in greater detail.
Figure 3 is similar to that of Figure 1 but showing the core wire in its extended position.
Figure 4 is similar to that of Figure 3 but showing the core wire in its retracted position.
Figure 5 is a view similar to that of Figure 1 bit showing an alternative embodiment of a guidewire made in accordance with the present invention.
Figure 6 is a cross-sectional view of the guidewire shown in Figure 5 taken along line 6-6.

### DETAILED DESCRIPTION OF THE INVENTION

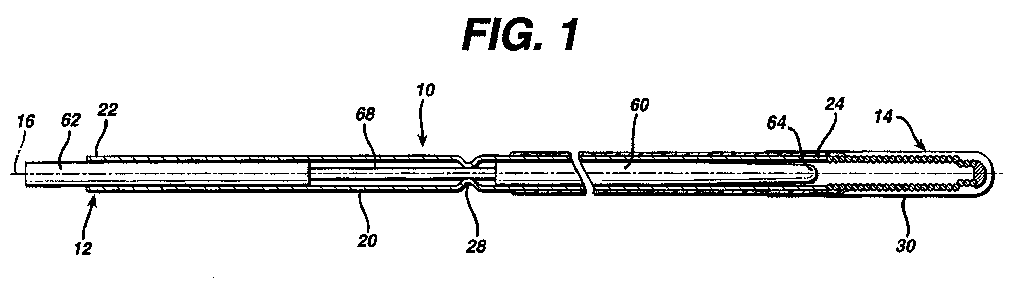

Referring to the drawings wherein like numerals indicate the same elements throughout the views, there is shown in Figure 1 a guidewire 10 made in accordance with the present invention. Guidewire 10 is a steerable percutaneous transluminal coronary angioplasty (PTCA) guidewire such as those described in U.S. Patent 5,267,574 issued to Viera et al. on December 7, 1993 or U.S. Patent 4,846,186 issued to Box et al. on July 11, 1989, both of which are hereby incorporated herein by reference. Guidewire 10 is designed to navigate through body vessels so as to guide and deliver balloon catheters and the like. Guidewire 10 has a proximal end 12, a distal end 14 and a longitudinal axis 16 extending therebetween. Guidewire 10 includes an outer tube 20. Tube 20 has a proximal end 22 and a distal end 24. As discussed below, outer tube 20 is preferably made from a material exhibiting substantially no shape memory retention. Such materials are well known to those skilled in the art and include stainless steel, plastics, etc.

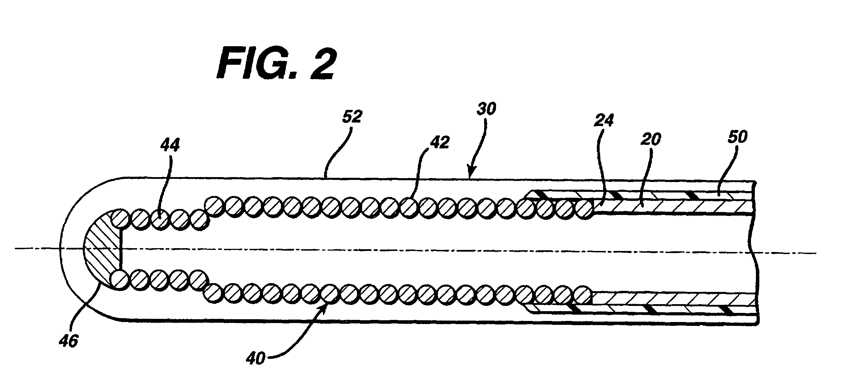

Guidewire 10 further includes a flexible distal tip 30, which can best be described by referring to Figure 2. Distal tip 20 is attached to distal end 24 of outer tube 20 and extends distally thereto. Flexible distal tip 30 is preferably includes a helical coil 40 and is attached to tube 20 by any means known to those skilled in the art including welding, soldering, adhesives, etc. Coil 40 is preferably made from a metal exhibiting shape memory retention such as Nitinol. This is so the coil will not permanently deform as it travels through the tortuous vasculature. Use of such materials in medical devices is described in U.S. Patent 5,067,957 issued to Jervis on November 26, 1991, which is hereby incorporated herein by reference. Coil 40 has a first uniform diameter region 42 and a second smaller uniform diameter region 44. This is so the tip 14 of the guidewire can accommodate a radiopaque marker band if desired. Coil 40 preferably includes a rounded tip weld 46 at the its proximal end. Coil 40 can also include radiopaque markers which are well known to those skilled in the art, or can be made from a radiopaque material.

Guidewire 10 includes a polytetrafluroethylene (PTFE) sleeve 50 which is heat shrunk over most of the length of tube 20 and extends distally to cover a portion of the coil 40. Sleeve 50 helps to connect coil 40 to tube 20. The distal end 40 is preferably coated with a hydrophilic coating 52. Distal end 40 may include a polymer coating under the hydrophilic coating.

By referring back to Figure 1, it can be shown that guidewire 10 further includes a core wire 60. Core wire 60 has distal end 64 and proximal end 62. The distal end 64 of core wire 60 is preferably made from a material exhibiting shape memory retention such as Nitinol. The proximal end 62 need not be made from a shape memory alloy, which can reduce the cost. Core wire 60 is disposed within tube 20 such that it can slide longitudinally within said tube. Preferably the distal tip 64 of the core wire is tapered down to a smaller diameter.

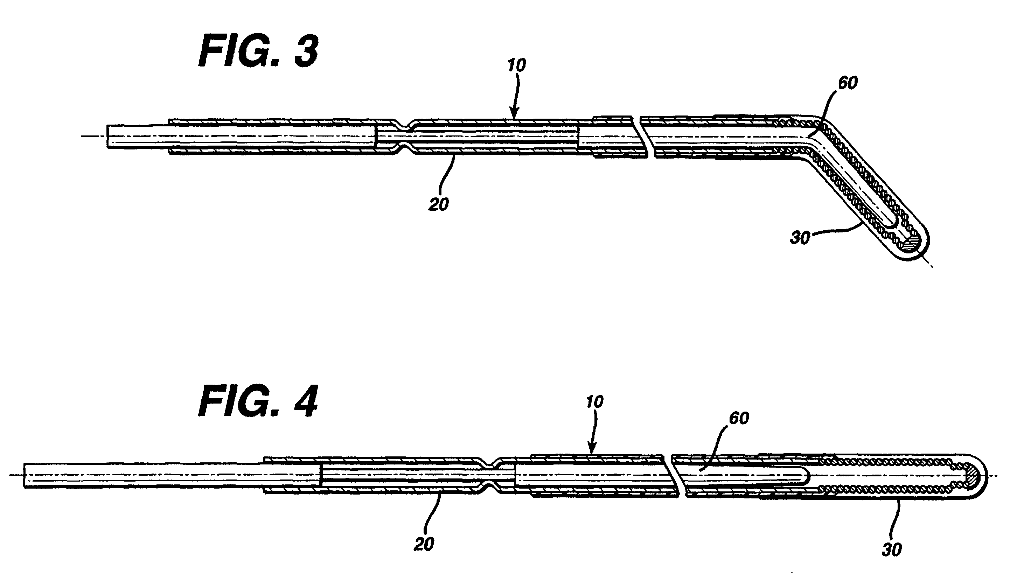

The core wire slides between a retracted and extended position. In the retracted position, the distal end of the core wire is proximal to the flexible distal tip, as shown in Figures 1, 2 and 4. In the retracted position, the distal end 64 of the core wire takes the shape of the outer tube 20. In the extended position, the distal end 64 of the core wire is distal to the distal end 24 of the outer tube, as shown in Figure 3. In the extended position core wire 60 can return to a preformed shape that was given to core wire prior to its insertion into the outer tube. Preferably the core wire can also rotate within the tube 20 as well. the force required to bend tube 50 is greater than the force required to bend the distal end 64 of the core wire.

Guidewire 10 can then be guided through the vasculature of a patient through a combination of extending, retracting and rotating the core wire 60. Extending the core gives the distal tip 30 of the guidewire a bent shape to help it make turns within the vasculature. There are numerous pre-bent shapes known to those skilled in the art which could be imparted to the distal end 64 of core wire 60.

Guidewire 10 preferably includes a means for preventing the removal of the core wire from the outer tube 20. As seen from figures 1,3 and 4, outer tube 20 includes a flange 28 and core wire 60 has a reduced diameter area 68. Area 68 allows the core wire 60 to slide within the tube and flange 28 prevents the core wire from being retracted too far.

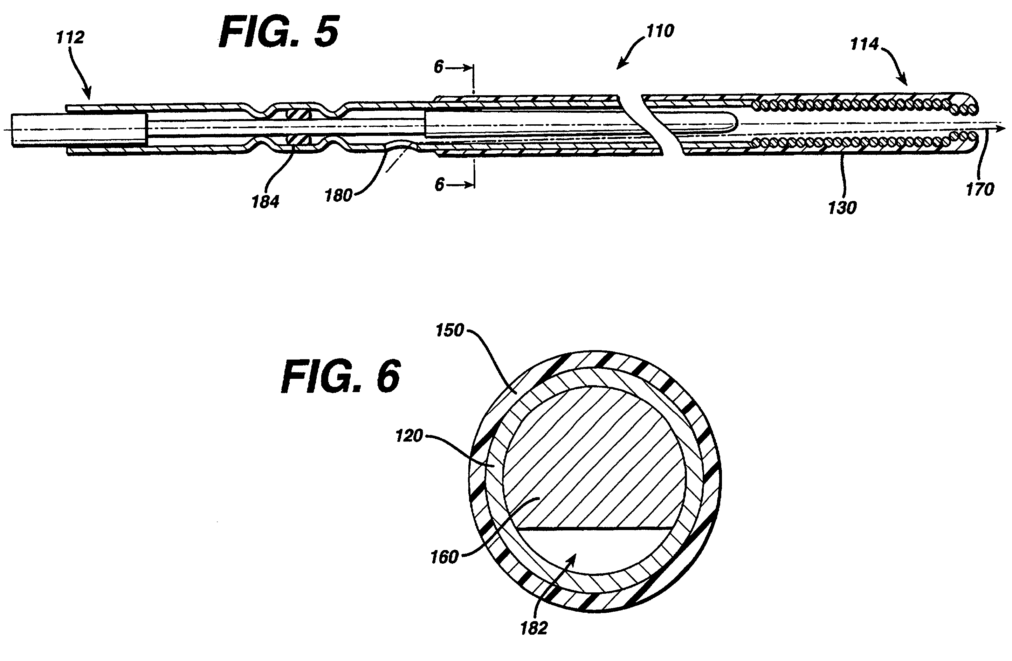

There is shown in Figure 5 a guidewire 110 which is an alternative embodiment of a guidewire made in accordance with the present invention. Guidewire 110 is similar to guidewire 10 except that it includes an injection port 180 at proximal end 112. Injection port 180 allows fluids, such as contrast medium, to be injected through the guidewire and into the vasculature of a patient. The core wire proximal to the injection port must allow for the passage of fluid through the distal end 114 of guidewire 110. As seen from Figure 6, guidewire 110 preferably includes a heat shrunk PTFE sleeve 150 over outer tube 120. Core wire 160 has a semicircular cross section so as to create a channel or lumen 182 for passage of fluid therethrough. Guidewire 110 preferably includes a seal 184, made from any suitable material such as rubber, to prevent fluid from being delivered through the proximal end of the catheter. The distal tip 130 of catheter 110 would be open so as to allow for the passage of fluid in the direction of arrow 170.

Although particular embodiments of the present invention have been shown and described, modification may be made to the catheter without departing from the scope of the present invention.

## Claims

1. A guidewire (10) for navigating through body vessels, said guidewire (10) having a proximal end (12), a distal end (14) and a longitudinal axis (16) extending therebetween, said guidewire (10) comprising:
a) an outer tube (20) having proximal and distal ends (22, 24), said outer tube (20) is made from a material exhibiting substantially no shape memory retention;
b) a flexible distal tip (30) attached to said distal end (24) of said outer tube (20) and extending distally thereto; and
c) a core wire (60) having distal and proximal ends (62, 64) said distal end (64) of said core wire (60) is made from a material exhibiting shape memory retention, said core wire (60) can slide longitudinally within said tube (20) between a retracted position, where said distal end (64) of said core wire (60) is proximal to said flexible distal tip (30) and takes the shape of said outer tube (20), and an extended position, wherein said distal end (64) of said core wire (60) is distal to said distal end (24) of said outer tube (20) so that it can return to a preformed shape.

2. The guidewire (10) according to claim 1 wherein said distal end (64) of said core wire (60) has a preformed shape which is at an angle to said longitudinal axis (16) of said guidewire (10).

3. The guidewire (10) according to claim 1 or 2, wherein said distal end (64) of said core wire (60) comprises Nitinol.

4. The guidewire (10) according to claim 1, 2 or 3, wherein said distal end (64) of said guidewire (10) tapers distally.

5. The guidewire (10) according to any one of claims 1 to 4 further including a means (68, 28) for preventing the removal of said core wire (60) from said tube (20).

6. The guidewire (10) according to any one of claims 1 to 5 wherein said flexible distal tip (30) of said guidewire (10) comprises a coiled spring.

7. The guidewire (10) according to Claim 6 wherein said coiled spring is made from a material substantially exhibiting shape memory retention.

8. The guidewire (10) according to claim 7 wherein said coiled spring comprises Nitinol.

9. The guidewire (110) according to any one of the preceding claims, comprising an injection part (180), the core wire (160) proximal to the injection part (180) allowing for the passage of fluid though the distal end (114) of the guidewire.

10. The guidewire (110) according to claim 9 wherein the core wire has a semicircular cross section.

## Patentansprüche

1. Führungsdraht (10) zum Finden des Weges durch Körpergefäße, wobei dieser Führungsdraht (10) ein proximales Ende (12), ein distales Ende (14) sowie eine sich zwischen beiden erstreckende Längsachse (16) hat und umfaßt:
a) ein Außenrohr (20) mit proximalem und distalem Ende (22, 24), welches aus einem Material hergestellt ist, das im wesentlichen keine Formgedächtnis-Remanenz aufweist;
b) eine flexible distale Spitze (30), die am distalen Ende (24) des Außenrohres (20) angebracht ist und sich von dort in distaler Richtung und
c) einen Kerndraht (60) mit distalem und proximalem Ende (62, 64), wobei dieser Kerndraht (60) aus einem Material mit Formgedächtnis-Remanenz hergestellt ist und in dem Rohr (20) zwischen einer zurückgezogenen Position, in welcher sich das distale Ende (64) des Kerndrahtes proximal von der flexiblen Spitze (30) befindet und die Form des Außenrohres (20) annimmt und einer ausgefahrenen Position in Längsrichtung gleiten kann, in welcher sich das distale Ende (64) des Kerndrahtes (60) distal vom distalen Ende (24) des Außenrohres (20) befindet, so daß das distale Ende des Kerndrahtes (60) eine vorgeformte Form wieder annehmen kann.

2. Führungsdraht (10) nach Anspruch 1, bei welchem das distale Ende (64) des Kerndrahtes (60) eine vorgeformte Form aufweist, welche unter einem Winkel zur Längsachse (16) des Führungsdrahtes (10) verläuft.

3. Führungsdraht (10) nach Anspruch 1 oder 2, bei welchem das distale Ende (64) des Kemdrahtes (60) aus Nitinol besteht.

4. Führungsdraht (10) nach Anspruch 1, 2 oder 3, bei welchem das distale Ende (64) des Führungsdrahtes (10) in distaler Richtung abgeschrägt ist.

5. Führungsdraht (10) nach einem der Ansprüch 1 bis 4, welcher weiterhin eine Einrichtung (68, 28) aufweist, um die Entnahme des Kerndrahtes (60) aus dem Rohr (20) zu verhindern.

6. Führungsdraht (10) nach einem der Ansprüche 1 bis 5, bei welchem die flexible Spitze (30) des Führungsdrahtes (10) aus einer Schraubenfeder besteht.

7. Führungsdraht (10) nach Anspruch 6, bei welchem die Schraubenfeder aus einem Material besteht, welches im wesentlichen Formgedächtnis-Remanenz aufweist.

8. Führungsdraht (10) nach Anspruch 7, bei welchem die Schraubenfeder aus Nitinol besteht.

9. Führungsdraht (10) nach einem der bisherigen Ansprüche mit einer Injektionsöffnung (180), wobei der Kerndraht (160) proximal zur Injektionsöffnung (180) den Durchtritt eines Fluids durch das distale Ende (114) des Führungsdrahtes (10) gestattet.

10. Führungsdraht (10) nach Anspruch 9, bei welchem der Kerndraht einen halbrunden Querschnitt hat.

## Revendications

1. Un fil de guidage (10) destiné à être introduit dans les vaisseaux anatomiques pour les parcourir, ledit fil de guidage (10) présentant une extrémité proximale (12) et une extrémité distale (14) ainsi qu'un axe longitudinal (16) s'étendant entre ces deux extrémités, ledit fil de guidage comportant :
a) un tube externe (20) présentant une extrémité proximale (22) et une extrémité distale (24), ledit tube externe (20) étant fait d'un matériau n'étant doté d'aucune propriété de mémoire de forme;
b) une terminaison flexible (30) solidaire de la dite extrémité distale (24) dudit tube externe (20) et s'éloignant de ladite extrémité distale (24); et
c) une âme (60) présentant une extrémité proximale (62) et une extrémité distale (64), ladite extrémité distale (64) de ladite âme (60) étant faite d'un matériau à mémoire de forme, ladite âme (60) étant à même de coulisser longitudinalement dans ledit tube externe (20) depuis une position rétractée, où ladite extrémité distale (64) de ladite âme (60) est proximale de ladite extrémité distale (24) dudit tube externe (20), point de fixation de ladite terminaison flexible (30) et prend la forme dudit tube externe (20), jusqu'à une position d'extension où ladite extrémité distale (64) de ladite âme (60) devient distale vis-à-vis de ladite extrémité distale (24) dudit tube externe (20) de façon à reprendre une forme prédéfinie.

2. Le fil de guidage (10) selon la revendication 1 dans lequel l'extrémité distale (64) de ladite âme (60) présente une forme prédéfinie, s'écartant angulairement dudit axe longitudinal (16) dudit fil de guidage (10).

3. le fil de guidage (10) selon l'une quelconque des revendications 1 ou 2, dans lequel ladite extrémité distale (64) dudit fil de guidage (60) se compose de Nitinol.

4. le fil de guidage (10) selon l'une quelconque des revendications 1, 2 ou 3, dans lequel ladite extrémité terminale (14) dudit fil de guidage (10) présente distalement une réduction de section.

5. Le fil de guidage (10) selon l'une quelconque des revendications 1 à 4, comportant de plus des moyens (68, 28) empêchant ladite âme (60) de coulisser hors dudit tube externe(20).

6. Le fil de guidage (10) selon l'une quelconque des revendications 1 à 5, dans lequel ladite terminaison flexible (30) dudit fil de guidage (10) comporte un ressort à boudin.

7. Le fil de guidage (10) selon la revendication 6 dans lequel ledit ressort à boudin est fait d'un matériau effectivement doté de propriétés de mémoire de forme.

8. Le fil de guidage (10) selon la revendication 7 dans lequel ledit ressort à boudin se compose de Nitinol.

9. Le fil de guidage (110) selon l'une quelconque des revendications 1 à 8, comportant une ouverture d'injection (180), la partie de l'âme (160) proximale de l'ouverture d'injection (180) permettant le passage d'un fluide à travers l'extrémité distale (114) du fil de guidage.

10. Le fil de guidage (110) selon la revendication 9 dans lequel l'âme présente une section semi-circulaire.
